# EUROPEAN PATENT SPECIFICATION

(11) **EP 1 824 784 B1**
(45) Date of publication and mention of the grant of the patent: **21.08.2019**
(21) Application number: 05823295.0
(22) Date of filing: 25.10.2005
(51) Int. Cl.: C01B 39/48, C01B 37/02, B01J 20/18, B01J 20/28, B01D 53/94, B01J 29/70, B01J 29/87, C07C 209/16, C07C 1/20

(54) **HIGH-SILICA MOLECULAR SIEVE CHA**
SILICIUMDIOXIDREICHES MOLSIEB CHA
TAMIS MOLECULAIRE A HAUTE TENEUR EN SILICE A STRUCTURE CHA

(30) Priority: 29.11.2004 US 631691 P; 29.11.2004 US 631834 P; 29.11.2004 US 631662 P; 29.11.2004 US 631726 P; 29.11.2004 US 631715 P
(43) Date of publication of application: 29.08.2007
(73) Proprietor: Chevron U.S.A. Inc., San Ramon, CA 94583 (US)
(72) Inventor: YUEN, Lun-Teh, San Francisco, CA 94132 (US)
(74) Representative: Haseltine Lake Kempner LLP
(86) International application number: PCT/US2005/038601
(87) International publication number: WO 2006/057760

(56) References cited:
- EP-A1- 0 532 803
- WO-A1-03/020641
- WO-A1-03/026780
- WO-A1-03/078324
- US-A- 4 496 786
- US-A- 4 544 538
- US-A- 4 544 538
- US-A1- 2003 069 449
- US-A1- 2003 089 227
- US-A1- 2003 176 751

## Description

### BACKGROUND

Chabazite, which has the crystal structure designated "CHA", is a natural zeolite with the approximate formula Ca₆Al₁₂Si₂₄O₇₂. Synthetic forms of chabazite are described in "Zeolite Molecular Sieves" by D.W. Breck, published in 1973 by John Wiley & Sons. The synthetic forms reported by Breck are: zeolite "K-G", described in J. Chem. Soc., p. 2822 (1956), Barrer et al.; zeolite D, described in British Patent No. 868,846 (1961); and zeolite R, described in U.S. Patent No. 3,030,181, issued April 17, 1962 to Milton. Chabazite is also discussed in "Atlas of Zeolite Structure Types" (1978) by W.H. Meier and D.H. Olson.

The K-G zeolite material reported in the J. Chem. Soc. Article by Barrer et al. is a potassium form having a silica:alumina mole ratio (referred to herein as "SAR") of 2.3:1 to 4.15:1. Zeolite D reported in British Patent No. 868,846 is a sodium-potassium form having a SAR of 4.5:1 to 4.9:1. Zeolite R reported in U.S. Patent No. 3,030,181 is a sodium form which has a SAR of 3.45:1 to 3.65:1.

Citation No. 93:66052y in Volume 93 (1980) of Chemical Abstracts concerns a Russian language article by Tsitsishrili et al. in Soobsch. Akad. Nauk. Gruz. SSR 1980, 97(3) 621-4. This article teaches that the presence of tetramethylammonium ions in a reaction mixture containing K₂O-Na₂O-SiO₂-Al₂O₃-H₂O promotes the crystallization of chabazite. The zeolite obtained by the crystallization procedure has a SAR of 4.23.

The molecular sieve designated SSZ-13, which has the CHA crystal structure, is disclosed in U.S. Patent No. 4,544,538, issued October 1, 1985 to Zones. SSZ-13 is prepared from nitrogen-containing cations derived from 1-adamantamine, 3-quinuclidinol and 2-exo-aminonorbornane. Zones discloses that the SSZ-13 of U.S. Patent No. 4,544,538 has a composition, as-synthesized and in the anhydrous state, in terms of mole ratios of oxides as follows:
(0.5 to 1.4)R₂O : (0 to 0.5)M₂O : W₂O₃: (greater than 5)YO₂
wherein M is an alkali metal cation, W is selected from aluminum, gallium and mixtures thereof, Y is selected from silicon, germanium and mixtures thereof, and R is an organic cation. As prepared, the silica:alumina mole ratio is typically in the range of 8:1 to about 50:1, higher mole ratios can be obtained by varying the relative ratios of reactants. It is disclosed that higher mole ratios can also be obtained by treating the SSZ-13 with chelating agents or acids to extract aluminum from the SSZ-13 lattice. It is further stated that the silica:alumina mole ratio can also be increased by using silicon and carbon halides and similar compounds.

U.S. Patent No. 4,544,538 also discloses that the reaction mixture used to prepare SSZ-13 has a YO₂/W₂O₃ mole ratio (e.g., SAR) in the range of 5:1 to 350:1. It is disclosed that use of an aqueous colloidal suspension of silica in the reaction mixture to provide a silica source allows production of SSZ-13 having a relatively high silica:alumina mole ratio.

U.S. Patent No. 4,544,538 does not, however, disclose SSZ-13 having a silica:alumina mole ratio greater than 50.

U.S. Patent No. 6,709,644, issued March 23, 2004 to Zones et al., discloses aluminosilicate zeolites having the CHA crystal structure and having small crystallite sizes (designated SSZ-62). The reaction mixture used to prepare SSZ-62 has a SiO₂/Al₂O₃ mole ratio of 20-50. It is disclosed that the zeolite can be used for separation of gasses (e.g., separating carbon dioxide from natural gas), and in catalysts used for the reduction of oxides of nitrogen in a gas stream (e.g., automotive exhaust), converting lower alcohols and other oxygenated hydrocarbons to liquid products, and for producing dimethylamine.

M.A. Camblor, L.A. Villaescusa and M. J. Diaz-Cabanas, "Synthesis of All-Silica and High-Silica Molecular Sieves in Fluoride Media", Topics in Catalysis, 9 (1999), pp. 59-76 discloses a method for making all-silica or high-silica zeolites, including chabazite. The chabazite is made in a reaction mixture containing fluoride and a N,N,N-trimethyl-1-adamantammonium structure directing agent. Camblor et al. does not, however, disclose the synthesis of all- or high-silica chabazite from a hydroxide-containing reaction mixture.

WO 03/078324 describes synthesis of a porous crystalline material having the structure of chabazite and having a composition involving the molar relationship X₂O₃:(n)YO₂, where n is greater than 100, the material is synthesized in a fluorine-containing medium.

### SUMMARY OF THE INVENTION

In accordance with this invention there is provided a method for preparing a molecular sieve having the CHA crystal structure and a mole ratio of greater than 200:1 of (1) silicon oxide, germanium oxide and mixtures thereof to (2) aluminum oxide, iron oxide, titanium oxide, gallium oxide and mixtures thereof, said method comprising:
A. forming an aqueous reaction mixture comprising a composition in terms of mole ratios falling within the following ranges:

| | |
|---|---|
| YO₂/WₐO_{b} | 350 - ∞ (preferably 350-5500) |
| OH-/YO₂ | 0.19-0.52 |
| Q/YO₂ | 0.15-0.25 |
| M_{2/n}O/YO₂ | 0.04-0.10 |
| H₂O/YO₂ | 10-50 |

wherein Y is silicon, germanium or mixtures thereof, W is aluminum, iron, titanium, gallium or mixtures thereof, a is 1 or 2 and b is 2 when a is 1 (i.e., W is tetravalent) or b is 3 when a is 2 (i.e., W is trivalent); M is an alkali metal or alkaline earth metal, n is the valence of M, and Q is a cation derived from 1-adamantamine, 3-quinuclidinol or 2-exo-aminonorbornane; and
B. maintaining said aqueous mixture under sufficient crystallization conditions until crystals are formed.

It should be noted that the reaction mixture does not contain fluorine. Thus, the molecular sieve is prepared in the absence of fluoride.

In accordance with this invention, there is also provided a high-silica molecular sieve having the CHA crystal structure and having a composition, as-synthesized and in the anhydrous state, in terms of mole ratios of oxides as follows:

| | |
|---|---|
| YO₂/W_{c}O_{d} | 200 -∞ (e.g., 200-1500) |
| M_{2/n}O/YO₂ | 0.04 - 0.15 |
| Q/YO₂ | 0.15 - 0.25 |

wherein Y is silicon, germanium or mixtures thereof, W is aluminum, iron, titanium, gallium or mixtures thereof; c is 1 or 2; d is 2 when c is 1 (i.e., W is tetravalent) or d is 3 or 5 when c is 2 (i.e., d is 3 when W is trivalent or 5 when W is pentavalent); M is an alkali metal cation, alkaline earth metal cation or mixtures thereof; n is the valence of M (i.e., 1 or 2); and Q is a cation derived from 1-adamantamine, 3-quinuclidinol or 2-exo-aminonorbornane. The as-synthesized material does not contain fluoride.

Also described herein is a molecular sieve having the CHA crystal structure and having a mole ratio of greater than 50 to 1000 of (1) an oxide selected from silicon oxide, germanium oxide or mixtures thereof to (2) an oxide selected from aluminum oxide, iron oxide, titanium oxide, gallium oxide or mixtures thereof. In one embodiment, the molecular sieve has a mole ratio of oxide (1) to oxide (2) is 200-1500.

Also described herein is a process for producing methylamine or dimethylamine comprising reacting methanol, dimethyl ether or a mixture thereof and ammonia in the gaseous phase in the presence of a catalyst comprising a molecular sieve having the CHA crystal structure and having a mole ratio of greater than 50 to 1500 of (1) an oxide selected from silicon oxide, germanium oxide or mixtures thereof to (2) an oxide selected from aluminum oxide, iron oxide, titanium oxide, gallium oxide or mixtures thereof. In one embodiment, the molecular sieve has a mole ratio of oxide (1) to oxide (2) is 200-1500.

Also described herein is process for the production of light olefins comprising olefins having from 2 to 4 carbon atoms per molecule from an oxygenate feedstock. The process comprises passing the oxygenate feedstock to an oxygenate conversion zone containing a molecular sieve catalyst to produce a light olefin stream.

Also described herein is a process for the production of light olefins from a feedstock comprising an oxygenate or mixture of oxygenates, the process comprising reacting the feedstock at effective conditions over a catalyst comprising a molecular sieve having the CHA crystal structure and having a mole ratio of greater than 50 to 1500 of (1) an oxide selected from silicon oxide, germanium oxide or mixtures thereof to (2) an oxide selected from aluminum oxide, iron oxide, titanium oxide, gallium oxide or mixtures thereof. In one embodiment, thee mole ratio of oxide (1) to oxide (2) is 200-1500.

Also described herein is an improved process for separating gasses using a membrane containing a molecular sieve, the improvement comprising using as the molecular sieve a molecular sieve having the CHA crystal structure and having a mole ratio of greater than 50 to 1500 of (1) an oxide selected from silicon oxide, germanium oxide or mixtures thereof to (2) an oxide selected from aluminum oxide, iron oxide, titanium oxide, gallium oxide or mixtures thereof. In one embodiment, the molecular sieve has a mole ratio of oxide (1) to oxide (2) is 200-1500.

Also described herein is a process for the reduction of oxides of nitrogen contained in a gas stream wherein said process comprises contacting the gas stream with a molecular sieve, the molecular sieve having the CHA crystal structure and having a mole ratio of greater than 50 to 1500 of (1) an oxide selected from silicon oxide, germanium oxide or mixtures thereof to (2) an oxide selected from aluminum oxide, iron oxide, titanium oxide, gallium oxide or mixtures thereof. In one embodiment, the molecular sieve has a mole ratio of oxide (1) to oxide (2) is 200-1500. The molecular sieve may contain a metal or metal ions (such as cobalt, copper, platinum, iron, chromium, manganese, nickel, zinc, lanthanum, palladium, rhodium or mixtures thereof) capable of catalyzing the reduction of the oxides of nitrogen, and the process may be conducted in the presence of a stoichiometric excess of oxygen. In a preferred embodiment, the gas stream is the exhaust stream of an internal combustion engine.

Also described herein is a process for treating an engine exhaust stream and in particular to a process for minimizing emissions during the cold start operation of an engine. Accordingly, described herein is
a process for treating a cold-start engine exhaust gas stream containing hydrocarbons and other pollutants consisting of flowing said engine exhaust gas stream over a molecular sieve bed which preferentially adsorbs the hydrocarbons over water to provide a first exhaust stream, and flowing the first exhaust gas stream over a catalyst to convert any residual hydrocarbons and other pollutants contained in the first exhaust gas stream to innocuous products and provide a treated exhaust stream and discharging the treated exhaust stream into the atmosphere, the molecular sieve bed characterized in that it comprises a molecular sieve having the CHA crystal structure and having a mole ratio of greater than 50 to 1000 of (1) an oxide selected from silicon oxide, germanium oxide or mixtures thereof to (2) an oxide selected from aluminum oxide, iron oxide, titanium oxide, gallium oxide or mixtures thereof. In one embodiment, the molecular sieve has a mole ratio of oxide (1) to oxide (2) is 200-1500.

Also described herein is a process wherein the engine is an internal combustion engine, including automobile engines, which can be fueled by a hydrocarbonaceous fuel.

Also described herein is a process wherein the molecular sieve has deposited on it a metal selected from the group consisting of platinum, palladium, rhodium, ruthenium, and mixtures thereof.

### DETAILED DESCRIPTION

The present invention relates to a method of preparing high-silica molecular sieves having the CHA crystal structure and the molecular sieves so prepared, as set out in claims 1 and 8. As used herein, the term "high-silica" means the molecular sieve has a mole ratio of (1) silicon oxide, germanium oxide and mixtures thereof to (2) aluminum oxide, iron oxide, titanium oxide, gallium oxide and mixtures thereof of greater than 50. This includes all-silica molecular sieves in which the ratio of (1):(2) is infinity, i.e., there is essentially none of oxide (2) in the molecular sieve.

One advantage of the present invention is that the reaction is conducted in the presence of hydroxide rather than fluoride. HF-based syntheses generally require a large amount of structure directing agent ("SDA"). Typical HF-based reactions will have a SDA/SiO₂ mole ratio of 0.5.

High-silica CHA molecular sieves can be suitably prepared from an aqueous reaction mixture containing sources of an alkali metal or alkaline earth metal oxide; sources of an oxide of silicon, germanium or mixtures thereof; optionally, sources of aluminum oxide, iron oxide, titanium oxide, gallium oxide and mixtures thereof; and a cation derived from 1-adamantamine, 3-quinuclidinol or 2-exo-aminonorbornane. The mixture should have a composition in terms of mole ratios falling within the ranges shown in Table A below:

**TABLE A**

| | |
|---|---|
| YO₂/WₐO_{b} | 350 - ∞ (preferably 350-5500) |
| OH-/YO₂ | 0.19-0.52 |
| Q/YO₂ | 0.15-0.25 |
| M_{2/n}O/YO₂ | 0.04-0.10 |
| H₂O/YO₂ | 10-50 |

wherein Y is silicon, germanium or mixtures thereof, W is aluminum, iron, titanium, gallium or mixtures thereof, M is an alkali metal or alkaline earth metal, n is the valence of M (i.e., 1 or 2) and Q is a cation derived from 1-adamantamine, 3-quinuclidinol or 2-exo-aminonorbornane.

The cation derived from 1-adamantamine can be a N,N,N-trialkyl-1-adamantammonium cation which has the formula: 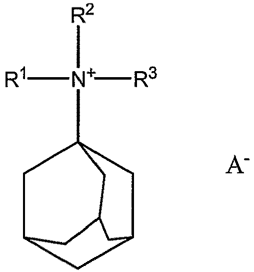 where R¹, R², and R³ are each independently a lower alkyl, for example methyl. The cation is associated with an anion, A⁻, which is not detrimental to the formation of the molecular sieve. Representative of such anions include halogens, such as chloride, bromide and iodide; hydroxide; acetate; sulfate and carboxylate. Hydroxide is the preferred anion. It may be beneficial to ion exchange, for example, a halide for hydroxide ion, thereby reducing or eliminating the alkali metal or alkaline earth metal hydroxide required.

The cation derived from 3-quinuclidinol is a cation derived from:

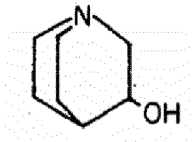

The cation derived from 2-exo-aminonorbornane can have the formula: 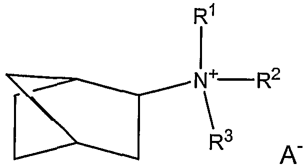 where R¹, R², R³ and A are as defined above.

The reaction mixture is prepared using standard molecular sieve preparation techniques. Typical sources of silicon oxide include fumed silica, silicates, silica hydrogel, silicic acid , colloidal silica, tetra-alkyl orthosilicates, and silica hydroxides. Examples of such silica sources include CAB-O-SIL M5 fumed silica and Hi-Sil hydrated amorphous silica, or mixtures thereof. Typical sources of aluminum oxide include aluminates, alumina, hydrated aluminum hydroxides, and aluminum compounds such as AlCl₃ and Al₂(SO₄)₃. Sources of other oxides are analogous to those for silicon oxide and aluminum oxide.

It has been found that seeding the reaction mixture with CHA crystals both directs and accelerates the crystallization, as well as minimizing the formation of undesired contaminants. In order to produce pure phase high-silica CHA crystals, seeding may be required. When seeds are used, they can be used in an amount that is about 2-3 wt.% based on the weight of YO₂.

The reaction mixture is maintained at an elevated temperature until CHA crystals are formed. The temperatures during the hydrothermal crystallization step are typically maintained from about 120°C to about 160°C. It has been found that a temperature below 160°C, e.g., about 120°C to about 140°C, is useful for producing high-silica CHA crystals without the formation of secondary crystal phases.

In one embodiment, the reaction mixture contains seeds of CHA crystals and the reaction mixture is maintained at a temperature of less than 160°C, for example 120°C to 140°C.

The crystallization period is typically greater than 1 day and preferably from about 3 days to about 7 days. The hydrothermal crystallization is conducted under pressure and usually in an autoclave so that the reaction mixture is subject to autogenous pressure. The reaction mixture can be stirred, such as by rotating the reaction vessel, during crystallization.

Once the high-silica CHA crystals have formed, the solid product is separated from the reaction mixture by standard mechanical separation techniques such as filtration. The crystals are water-washed and then dried, e.g., at 90°C to 150°C for from 8 to 24 hours, to obtain the as-synthesized crystals. The drying step can be performed at atmospheric or subatmospheric pressures.

The high-silica CHA can be made with a mole ratio of YO₂/W_{c}O_{d} of ∞, i.e., there is essentially no W_{c}O_{d} present in the CHA. In this case, the CHA would be an all-silica material or a germanosilicate. Thus, in a typical case where oxides of silicon and aluminum are used, CHA can be made essentially aluminum free, i.e., having a silica to alumina mole ratio of ∞. A method of increasing the mole ratio of silica to alumina is by using standard acid leaching or chelating treatments. The high-silica CHA can also be made by first preparing a borosilicate CHA and then removing the boron. The boron can be removed by treating the borosilicate CHA with acetic acid at elevated temperature (as described in Jones et al., Chem. Mater., 2001, 13, pp. 1041-1050) to produce an all-silica version of CHA.

The high-silica CHA molecular sieve has a composition, as-synthesized and in the anhydrous state, in terms of mole ratios of oxides as indicated in Table B below:

**TABLE B**

| As-Synthesized High-Silica CHA Composition | |
|---|---|
| YO₂/W_{c}O_{d} | 200 -∞ (e.g., 200-1500) |
| M_{2/n}O/YO₂ | 0.04 - 0.15 |
| Q/YO₂ | 0.15 - 0.25 |

wherein Y is silicon, germanium or mixtures thereof, W is aluminum, iron, titanium, gallium or mixtures thereof; c is 1 or 2; d is 2 when c is 1 (i.e., W is tetravalent) or d is 3 or 5 when c is 2 (i.e., d is 3 when W is trivalent or 5 when W is pentavalent); M is an alkali metal cation, alkaline earth metal cation or mixtures thereof; n is the valence of M (i.e., 1 or 2); and Q is a cation derived from 1-adamantamine, 3-quinuclidinol or 2-exo-aminonorbornane. The as-synthesized material does not contain fluoride.

High-silica CHA molecular sieves can be used as-synthesized or can be thermally treated (calcined). By "thermal treatment" is meant heating to a temperature from about 200°C to about 820°C, either with or without the presence of steam. Usually, it is desirable to remove the alkali metal cation by ion exchange and replace it with hydrogen, ammonium, or any desired metal ion. Thermal treatment including steam helps to stabilize the crystalline lattice from attack by acids.

The high silica CHA molecular sieves, as-synthesized, have a crystalline structure whose X-ray powder diffraction ("XRD") pattern shows the following characteristic lines:

**TABLE I**

| As-Synthesized High Silica CHA XRD | | |
|---|---|---|
| 2 Theta^{(a)} | d-spacing (Angstroms) | Relative Intensity^{(b)} |
| 9.64 | 9.17 | S |
| 14.11 | 6.27 | M |
| 16.34 | 5.42 | VS |
| 17.86 | 4.96 | M |
| 21.03 | 4.22 | VS |
| 25.09 | 3.55 | S |
| 26.50 | 3.36 | W-M |
| 30.96 | 2.89 | W |
| 31.29 | 2.86 | M |
| 31.46 | 2.84 | W |

| | | |
|---|---|---|
| ^{(a)} ± 0.10 ^{(b)} The X-ray patterns provided are based on a relative intensity scale in which the strongest line in the X-ray pattern is assigned a value of 100: W(weak) is less than 20; M(medium) is between 20 and 40; S(strong) is between 40 and 60; VS(very strong) is greater than 60. | | |

Table IA below shows the X-ray powder diffraction lines for as-synthesized high silica CHA including actual relative intensities.

**TABLE IA**

| As-Synthesized High Silica CHA XRD | | |
|---|---|---|
| 2 Theta^{(a)} | d-spacing (Angstroms) | Relative Intensity(%) |
| 9.64 | 9.17 | 50.8 |
| 13.16 | 6.72 | 4.4 |
| 14.11 | 6.27 | 23.1 |
| 16.34 | 5.42 | 82.4 |
| 17.86 | 4.96 | 21.7 |
| 19.34 | 4.59 | 6.1 |
| 21.03 | 4.22 | 100 |
| 22.24 | 3.99 | 11.0 |
| 22.89 | 3.88 | 10.7 |
| 23.46 | 3.79 | 4.9 |
| 25.09 | 3.55 | 43.1 |
| 26.50 | 3.36 | 19.5 |
| 28.25 | 3.16 | 4.7 |
| 28.44 | 3.14 | 1.5 |
| 30.14 | 2.96 | 3.2 |
| 30.96 | 2.89 | 14.3 |
| 31.29 | 2.86 | 37.5 |
| 31.46 | 2.84 | 12.0 |
| 33.01 | 2.71 | 1.8 |
| 33.77 | 2.65 | 1.9 |
| 34.05 | 2.63 | 0.2 |
| 35.28 | 2.54 | 3.6 |
| 35.69 | 2.51 | 0.7 |
| 36.38 | 2.47 | 5.8 |
| 39.22 | 2.30 | 1.0 |
| 39.81 | 2.26 | 0.8 |

| | | |
|---|---|---|
| ^{(a)} ± 0.10 | | |

After calcination, the high silica CHA molecular sieves have a crystalline structure whose X-ray powder diffraction pattern include the characteristic lines shown in Table II:

**TABLE II**

| Calcined High Silica CHA XRD | | |
|---|---|---|
| 2 Theta^{(a)} | d-spacing (Angstroms) | Relative Intensity |
| 9.65 | 9.2 | VS |
| 13.08 | 6.76 | M |
| 16.28 | 5.44 | W |
| 18.08 | 4.90 | W |
| 20.95 | 4.24 | M |
| 25.37 | 3.51 | W |
| 26.36 | 3.38 | W |
| 31.14 | 2.87 | M |
| 31.61 | 2.83 | W |
| 35.10 | 2.55 | W |

| | | |
|---|---|---|
| ^{(a)} ± 0.10 | | |

Table IIA below shows the X-ray powder diffraction lines for calcined high silica CHA including actual relative intensities.

**TABLE IIA**

| Calcined High Silica CHA XRD | | |
|---|---|---|
| 2 Theta^{(a)} | d-spacing (Angstroms) | Relative Intensity(%) |
| 9.65 | 9.2 | 100 |
| 13.08 | 6.76 | 29.3 |
| 14.21 | 6.23 | 3.9 |
| 16.28 | 5.44 | 15.2 |
| 18.08 | 4.90 | 16.1 |
| 19.37 | 4.58 | 2.3 |
| 20.95 | 4.24 | 36.8 |
| 22.38 | 3.97 | 1.9 |
| 22.79 | 3.90 | 1.9 |
| 23.44 | 3.79 | 1.5 |
| 25.37 | 3.51 | 14.1 |
| 26.36 | 3.38 | 9.5 |
| 28.12 | 3.17 | 2.0 |
| 28.65 | 3.11 | 1.9 |
| 30.07 | 2.97 | 1.0 |
| 31.14 | 2.87 | 22.0 |
| 31.36 | 2.85 | 2.9 |
| 31.61 | 2.83 | 9.3 |
| 32.14 | 2.78 | 0.9 |
| 32.90 | 2.72 | 1.0 |
| 34.03 | 2.63 | 2.1 |
| 35.10 | 2.55 | 4.3 |
| 36.64 | 2.45 | 3.3 |
| 39.29 | 2.29 | 1.3 |
| 40.40 | 2.23 | 2.6 |

| | | |
|---|---|---|
| ^{(a)} ± 0.10 | | |

The X-ray powder diffraction patterns were determined by standard techniques. The radiation was the K-alpha/doublet of copper and a scintillation counter spectrometer with a strip-chart pen recorder was used. The peak heights I and the positions, as a function of 2 Theta where Theta is the Bragg angle, were read from the spectrometer chart. From these measured values, the relative intensities, 100 x I/Io, where Io is the intensity of the strongest line or peak, and d, the interplanar spacing in Angstroms corresponding to the recorded lines, can be calculated.

Variations in the diffraction pattern can result from variations in the mole ratio of oxides from sample to sample. The molecular sieve produced by exchanging the metal or other cations present in the molecular sieve with various other cations yields a similar diffraction pattern, although there can be shifts in interplanar spacing as well as variations in relative intensity. Calcination can also cause shifts in the X-ray diffraction pattern. Also, the symmetry can change based on the relative amounts of boron and aluminum in the crystal structure. Notwithstanding these perturbations, the basic crystal lattice structure remains unchanged.

The molecular sieve of the present invention can be used in a catalyst to prepare methylamine or dimethylamine. Dimethylamine is generally prepared in industrial quantities by continuous reaction of methanol (and/or dimethylether) and ammonia in the presence of a silica-alumina catalyst. The reactants are typically combined in the vapor phase, at temperatures in the range of 300°C to 500°C, and at elevated pressures. Such a process is disclosed in U. S. Patent No. 4,737,592, issued April 12, 1988 to Abrams et al.

The catalyst is used in its acid form. Acid forms of molecular sieves can be prepared by a variety of techniques. Preferably, the molecular sieve used to prepare dimethylamine will be in the hydrogen form, or have an alkali or alkaline earth metal, such as Na, K, Rb, or Cs, ion-exchanged into it.

The process of the present invention involves reacting methanol, dimethylether or a mixture thereof and ammonia in amounts sufficient to provide a carbon/nitrogen (C/N) ratio from about 0.2 to about 1.5, preferably about 0.5 to about 1.2. The reaction is conducted at a temperature from about 250°C to about 450°C, preferably about 300°C to about 400°C. Reaction pressures can vary from about 7-7000 kPa (1-1000 psi), preferably about 70-3000 kPa (10-500 psi). A methanol and/or dimethylether space time of about 0.01-80 hours, preferably 0.10-1.5 hours, is typically used. This space time is calculated as the mass of catalyst divided by the mass flow rate of methanol/dimethylether introduced into the reactor.

The present invention comprises a process for catalytic conversion of a feedstock comprising one or more oxygenates comprising alcohols and ethers to a hydrocarbon product containing light olefins, i.e., C₂, C₃ and/or C₄ olefins. The feedstock is contacted with the molecular sieve of the present invention at effective process conditions to produce light olefins.

The term "oxygenate" as used herein designates compounds such as alcohols, ethers and mixtures thereof. Examples of oxygenates include, but are not limited to, methanol and dimethyl ether.

The process of the present invention may be conducted in the presence of one or more diluents which may be present in the oxygenate feed in an amount between about 1 and about 99 molar percent, based on the total number of moles of all feed and diluent components. Diluents include, but are not limited to, helium, argon, nitrogen, carbon monoxide, carbon dioxide, hydrogen, water, paraffins, hydrocarbons (such as methane and the like), aromatic compounds, or mixtures thereof. U. S. Patents No. 4,861,938 and 4,677,242,
emphasize the use of a diluent to maintain catalyst selectivity toward the production of light olefins, particularly ethylene.

The oxygenate conversion is preferably conducted in the vapor phase such that the oxygenate feedstock is contacted in a vapor phase in a reaction zone with the molecular sieve of this invention at effective process conditions to produce hydrocarbons, i.e., an effective temperature, pressure, weight hourly space velocity (WHSV) and, optionally, an effective amount of diluent. The process is conducted for a period of time sufficient to produce the desired light olefins. In general, the residence time employed to produce the desired product can vary from seconds to a number of hours. It will be readily appreciated that the residence time will be determined to a significant extent by the reaction temperature , the molecular sieve catalyst, the WHSV, the phase (liquid or vapor) and process design characteristics. The oxygenate feedstock flow rate affects olefin production. Increasing the feedstock flow rate increases WHSV and enhances the formation of olefin production relative to paraffin production. However, the enhanced olefin production relative to paraffin production is offset by a diminished conversion of oxygenate to hydrocarbons.

The oxygenate conversion process is effectively carried out over a wide range of pressures, including autogenous pressures. At pressures between about 0.01 atmospheres (0.1 kPa) and about 1000 atmospheres (101.3 kPa), the formation of light olefins will be affected although the optimum amount of product will not necessarily be formed at all pressures. The preferred pressure is between about 0.01 atmospheres (0.1 kPa) and about 100 atmospheres (10.13 kPa). More preferably, the pressure will range from about 1 to about 10 atmospheres (101.3 kPa to 1.013 Mpa). The pressures referred to herein are exclusive of the diluent, if any, that is present and refer to the partial pressure of the feedstock as it relates to oxygenate compounds.

The temperature which may be employed in the oxygenate conversion process may vary over a wide range depending, at least in part, on the molecular sieve catalyst. In general, the process can be conducted at an effective temperature between about 200°C and about 700°C. At the lower end of the temperature range, and thus generally at a lower rate of reaction, the formation of the desired light olefins may become low. At the upper end of the range , the process may not form an optimum amount of light olefins and catalyst deactivation may be rapid.

The molecular sieve catalyst preferably is incorporated into solid particles in which the catalyst is present in an amount effective to promote the desired conversion of oxygenates to light olefins. In one aspect, the solid particles comprise a catalytically effective amount of the catalyst and at least one matrix material selected from the group consisting of binder materials, filler materials and mixtures thereof to provide a desired property or properties, e.g., desired catalyst dilution, mechanical strength and the like to the solid particles. Such matrix materials are often, to some extent, porous in nature and may or may not be effective to promote the desired reaction. Filler and binder materials include, for example, synthetic and naturally occurring substances such as metal oxides, clays, silicas, aluminas, silica-aluminas, silica-magnesias, silica-zirconias, silica-thorias and the like. If matrix materials are included in the catalyst composition, the molecular sieve preferably comprises about 1 to 99%, more preferably about 5 to 90%, and still more preferably about 10 to 80% by weight of the total composition.

The molecular sieve of the present invention can be used to separate gasses. For example, it can be used to separate carbon dioxide from natural gas. Typically, the molecular sieve is used as a component in a membrane that is used to separate the gasses. Examples of such membranes are disclosed in U.S. Patent No. 6,508,860, issued January 21, 2003 to Kulkarni et al.

The molecular sieves of this invention may be used for the catalytic reduction of the oxides of nitrogen in a gas stream. Typically, the gas stream also contains oxygen, often a stoichiometric excess thereof. Also, the molecular sieve may contain a metal or metal ions within or on it which are capable of catalyzing the reduction of the nitrogen oxides. Examples of such metals or metal ions include cobalt, copper, platinum, iron, chromium, manganese, nickel, zinc, lanthanum, palladium, rhodium and mixtures thereof.

One example of such a process for the catalytic reduction of oxides of nitrogen in the presence of a zeolite is disclosed in U.S. Patent No. 4,297,328, issued October 27, 1981 to Ritscher et al.
There, the catalytic process is the combustion of carbon monoxide and hydrocarbons and the catalytic reduction of the oxides of nitrogen contained in a gas stream, such as the exhaust gas from an internal combustion engine. The zeolite used is metal ion-exchanged, doped or loaded sufficiently so as to provide an effective amount of catalytic copper metal or copper ions within or on the zeolite. In addition, the process is conducted in an excess of oxidant, e.g., oxygen.

As stated this invention generally relates to a process for treating an engine exhaust stream and in particular to a process for minimizing emissions during the cold start operation of an engine. The engine consists of any internal or external combustion engine which generates an exhaust gas stream containing noxious components or pollutants including unburned or thermally degraded hydrocarbons or similar organics. Other noxious components usually present in the exhaust gas include nitrogen oxides and carbon monoxide. The engine may be fueled by a hydrocarbonaceous fuel. As used in this specification and in the appended claims, the term "hydrocarbonaceous fuel" includes hydrocarbons, alcohols and mixtures thereof. Examples of hydrocarbons which can be used to fuel the engine are the mixtures of hydrocarbons which make up gasoline or diesel fuel. The alcohols which may be used to fuel engines include ethanol and methanol. Mixtures of alcohols and mixtures of alcohols and hydrocarbons can also be used. The engine may be a jet engine, gas turbine, internal combustion engine, such as an automobile, truck or bus engine, a diesel engine or the like. The process of this invention is particularly suited for hydrocarbon, alcohol, or hydrocarbon-alcohol mixture, internal combustion engine mounted in an automobile. For convenience the description will use hydrocarbon as the fuel to exemplify the invention. The use of hydrocarbon in the subsequent description is not to be construed as limiting the invention to hydrocarbon fueled engines.

When the engine is started up, it produces a relatively high concentration of hydrocarbons in the engine exhaust gas stream as well as other pollutants. Pollutants will be used herein to collectively refer to any unburned fuel components and combustion byproducts found in the exhaust stream. For example, when the fuel is a hydrocarbon fuel, hydrocarbons, nitrogen oxides, carbon monoxide and other combustion byproducts will be found in the engine exhaust gas stream. The temperature of this engine exhaust stream is relatively cool, generally below 500° C. and typically in the range of 200° to 400° C. This engine exhaust stream has the above characteristics during the initial period of engine operation, typically for the first 30 to 120 seconds after startup of a cold engine. The engine exhaust stream will typically contain, by volume, about 500 to 1000 ppm hydrocarbons.

The engine exhaust gas stream which is to be treated is flowed over a molecular sieve bed comprising the molecular sieve of this invention to produce a first exhaust stream. The molecular sieve is described below. The first exhaust stream which is discharged from the molecular sieve bed is now flowed over a catalyst to convert the pollutants contained in the first exhaust stream to innocuous components and provide a treated exhaust stream which is discharged into the atmosphere. It is understood that prior to discharge into the atmosphere, the treated exhaust stream may be flowed through a muffler or other sound reduction apparatus well known in the art.

The catalyst which is used to convert the pollutants to innocuous components is usually referred to in the art as a three-component control catalyst because it can simultaneously oxidize any residual hydrocarbons present in the first exhaust stream to carbon dioxide and water, oxidize any residual carbon monoxide to carbon dioxide and reduce any residual nitric oxide to nitrogen and oxygen. In some cases the catalyst may not be required to convert nitric oxide to nitrogen and oxygen, e.g., when an alcohol is used as the fuel. In this case the catalyst is called an oxidation catalyst. Because of the relatively low temperature of the engine exhaust stream and the first exhaust stream, this catalyst does not function at a very high efficiency, thereby necessitating the molecular sieve bed.

When the molecular sieve bed reaches a sufficient temperature, typically about 150-200° C., the pollutants which are adsorbed in the bed begin to desorb and are carried by the first exhaust stream over the catalyst. At this point the catalyst has reached its operating temperature and is therefore capable of fully converting the pollutants to innocuous components.

The adsorbent bed used in the instant invention can be conveniently employed in particulate form or the adsorbent can be deposited onto a solid monolithic carrier. When particulate form is desired, the adsorbent can be formed into shapes such as pills, pellets, granules, rings, spheres, etc. In the employment of a monolithic form, it is usually most convenient to employ the adsorbent as a thin film or coating deposited on an inert carrier material which provides the structural support for the adsorbent. The inert carrier material can be any refractory material such as ceramic or metallic materials. It is desirable that the carrier material be unreactive with the adsorbent and not be degraded by the gas to which it is exposed. Examples of suitable ceramic materials include sillimaite, petalite, cordierite, mullite, zircon, zircon mullite, spondumene, alumina-titanate, etc. Additionally, metallic materials which are within the scope of this invention include metals and alloys as disclosed in U.S. Pat. No. 3,920,583 which are oxidation resistant and are otherwise capable of withstanding high temperatures.

The carrier material can best be utilized in any rigid unitary configuration which provides a plurality of pores or channels extending in the direction of gas flow. It is preferred that the configuration be a honeycomb configuration.

The honeycomb structure can be used advantageously in either unitary form, or as an arrangement of multiple modules. The honeycomb structure is usually oriented such that gas flow is generally in the same direction as the cells or channels of the honeycomb structure. For a more detailed discussion of monolithic structures, refer to U.S. Pat. Nos. 3,785,998 and 3,767,453.

The molecular sieve is deposited onto the carrier by any convenient way well known in the art. A preferred method involves preparing a slurry using the molecular sieve and coating the monolithic honeycomb carrier with the slurry. The slurry can be prepared by means known in the art such as combining the appropriate amount of the molecular sieve and a binder with water. This mixture is then blended by using means such as sonification, milling, etc. This slurry is used to coat a monolithic honeycomb by dipping the honeycomb into the slurry, removing the excess slurry by draining or blowing out the channels, and heating to about 100° C. If the desired loading of molecular sieve is not achieved, the above process may be repeated as many times as required to achieve the desired loading.

Instead of depositing the molecular sieve onto a monolithic honeycomb structure, one can take the molecular sieve and form it into a monolithic honeycomb structure by means known in the art.

The adsorbent may optionally contain one or more catalytic metals dispersed thereon. The metals which can be dispersed on the adsorbent are the noble metals which consist of platinum, palladium, rhodium, ruthenium, and mixtures thereof. The desired noble metal may be deposited onto the adsorbent, which acts as a support, in any suitable manner well known in the art. One example of a method of dispersing the noble metal onto the adsorbent support involves impregnating the adsorbent support with an aqueous solution of a decomposable compound of the desired noble metal or metals, drying the adsorbent which has the noble metal compound dispersed on it and then calcining in air at a temperature of about 400° to about 500° C. for a time of about 1 to about 4 hours. By decomposable compound is meant a compound which upon heating in air gives the metal or metal oxide. Examples of the decomposable compounds which can be used are set forth in U.S. Pat. No. 4,791,091 . Preferred decomposable compounds are chloroplatinic acid, rhodium trichloride, chloropalladic acid, hexachloroiridate (IV) acid and hexachlororuthenate. It is preferable that the noble metal be present in an amount ranging from about 0.01 to about 4 weight percent of the adsorbent support. Specifically, in the case of platinum and palladium the range is 0.1 to 4 weight percent, while in the case of rhodium and ruthenium the range is from about 0.01 to 2 weight percent.

These catalytic metals are capable of oxidizing the hydrocarbon and carbon monoxide and reducing the nitric oxide components to innocuous products. Accordingly, the adsorbent bed can act both as an adsorbent and as a catalyst.

The catalyst which is used in this invention is selected from any three component control or oxidation catalyst well known in the art. Examples of catalysts are those described in U.S. Pat. Nos. 4,528,279; 4,791,091; 4,760,044; 4,868,148; and 4,868,149.
Preferred catalysts well known in the art are those that contain platinum and rhodium and optionally palladium, while oxidation catalysts usually do not contain rhodium. Oxidation catalysts usually contain platinum and/or palladium metal. These catalysts may also contain promoters and stabilizers such as barium, cerium, lanthanum, nickel, and iron. The noble metals promoters and stabilizers are usually deposited on a support such as alumina, silica, titania, zirconia, aluminosilicates, and mixtures thereof with alumina being preferred. The catalyst can be conveniently employed in particulate form or the catalytic composite can be deposited on a solid monolithic carrier with a monolithic carrier being preferred. The particulate form and monolithic form of the catalyst are prepared as described for the adsorbent above.

### EXAMPLES

### Examples 1, 2, 5, 7-16 and Reference Examples 3, 4 and 6

High silica CHA is synthesized by preparing the gel compositions, i.e., reaction mixtures, having the compositions, in terms of mole ratios, shown in the table below. The resulting gel is placed in a Parr bomb reactor and heated in an oven at the temperature indicated below while rotating at the speed indicated below. Products are analyzed by X-ray diffraction (XRD) and found to be high silica molecular sieves having the CHA structure. The source of silicon oxide is Cabosil M-5 fumed silica or HiSil 233 amorphous silica (0.208 wt.% alumina). The source of aluminum oxide is Reheis F 2000 alumina.

| | Ex. No. | SiO₂/ Al₂O₃ | OH- / SiO₂ | SDA¹/ SiO₂ | Na+ / SiO₂ | H₂O/ SiO₂ | Wt.% Seed | Rxn. Cond.² | Yield (g) | Product Actual SiO₂/Al₂O ₃ | Product Estimated SiO₂/Al₂O ₃ |
|---|---|---|---|---|---|---|---|---|---|---|---|
| | 1 | 1,731⁴ | 0.34 | 0.18 | 0.16 | 15.62 | 4.12 | 120/43/6 | 0.08 | | 95 |
| | 2 | 1,907 | 0.36 | 0.18 | 0.19 | 15.68 | 4.12 | 120/43/8 | 0.10 | | 131 |
| ***Ref.** | 3 | 224³ | 0.19 | 0.18 | 0.01 | 16.59 | 4.02 | 120/43/7 | 13.39 | 166 | |
| ***Ref.** | 4 | 221³ | 0.36 | 0.18 | 0.18 | 16.16 | 4.15 | 120/43/7 | 1.29 | 167 | |
| | 5 | 2,485⁴ | 0.36 | 0.18 | 0.18 | 16.03 | 4.12 | 120/43/7 | 0.11 | | 188 |
| ***Ref.** | 6 | 296⁴ | 0.37 | 0.18 | 0.19 | 15.84 | 4.16 | 120/43/6 | 0.98 | | 201 |
| | 7 | 1,731 | 0.36 | 0.18 | 0.19 | 15.68 | 4.12 | 120/43/5 | 0.18 | | 214 |
| | 8 | 407⁴ | 0.40 | 0.21 | 0.19 | 44.39 | 2.01 | 160/43/4 | 0.53 | | 290 |
| | 9 | 435 | 0.42 | 0.21 | 0.21 | 45.81 | 4.02 | 150/100/4 | 15.03 | 296 | |
| | 10 | 982⁴ | 0.42 | 0.31 | 0.11 | 28.03 | 2.78 | 140/43/5 | 0.38 | | 346 |
| | 11 | 350³ | 0.36 | 0.18 | 0.18 | 16.16 | 4.15 | 120/43/5 | 1.43 | | 347 |
| | 12 | 1,731⁴ | 0.36 | 0.18 | 0.19 | 15.68 | 4.12 | 12C/43/6 | 0.33 | 584 | |
| | 13 | 980⁴ | 0.33 | 0.25 | 0.08 | 22.70 | 2.78 | 140/43/5 | 0.92 | | 628 |
| | 14 | 4,135 | 0.36 | 0.17 | 0.19 | 15.86 | 5.01 | 120/200/5 | 6.90 | 682 | |
| | 15 | 5,234 | 0.33 | 0.15 | 0.18 | 11.62 | 4.7 | 120/43/4 | 0.3 | | 783 |
| | 16 | 4,104 | 0.37 | 0.18 | 0.19 | 18.11 | 5.01 | 120/75/5 | 7.37 | 1,394 | |

| | | | | | | | | | | | |
|---|---|---|---|---|---|---|---|---|---|---|---|
| ¹SDA = Cation derived from 1-adamantamine ²°C/RPM/Days ³SiO₂ source = Hi Sil ⁴SiO₂ source = CAB-O-SIL The product of each reaction is a crystalline molecular sieve having the CHA structure. *Reference Examples 3, 4 and 6 fall outside the scope of claim 1. | | | | | | | | | | | |

## Claims

1. A method for preparing a molecular sieve having the CHA crystal structure and a mole ratio of greater than 200:1 of (1) silicon oxide, germanium oxide and mixtures thereof to (2) aluminum oxide, iron oxide, titanium oxide, gallium oxide and mixtures thereof said method comprising:
A. forming an aqueous reaction mixture comprising a composition in terms of mole ratios falling within the following ranges:
| | |
|---|---|
| YO₂/WₐO_{b} | 350 - ∞ |
| OH-/YO₂ | 0.19-0.52 |
| Q/YO₂ | 0.15-0.25 |
| M_{2/n}O/YO₂ | 0.04-0.10 |
| H₂O/YO₂ | 10-50 |
wherein Y is silicon, germanium or mixtures thereof, W is aluminum, iron, titanium, gallium or mixtures thereof, a is 1 or 2, b is 2 when a is 1 or b is 3 when a is 2; M is an alkali metal or alkaline earth metal, n is the valence of M, and Q is a cation derived from 1-adamantamine, 3-quinuclidinol or 2-exo-aminonorbornane; and
B. maintaining said aqueous mixture under sufficient crystallization conditions until crystals are formed,
wherein the molecular sieve is prepared in the absence of fluorine.

2. The method of claim 1 wherein the reaction mixture further comprises seeds of a molecular sieve having the CHA structure.

3. The method of claim 1 wherein the reaction mixture is heated at a temperature of about 120 °C to about 160 °C.

4. The method of claim 3 wherein the reaction mixture is heated to a temperature of about 120 °C to about 140 °C.

5. The method of claim 2 wherein the reaction mixture is heated to a temperature of about 120 °C to about 140 °C.

6. The method according to any of claims 1 to 5, wherein the mole ratio of oxide (1) to oxide (2) is 200-1500.

7. The method according to any of claims 1 to 6, wherein the mole ratio of oxide (1) to oxide (2) is infinity.

8. A molecular sieve having the CHA crystal structure and having a composition, as-synthesized and in the anhydrous state, in terms of mole ratios of oxides as follows:
| | |
|---|---|
| YO₂/W_{c}O_{d} | 200 - ∞ |
| M_{2/n}O/YO₂ | 0.04-0.15 |
| Q/YO₂ | 0.15-0.25 |
wherein Y is silicon, germanium or mixtures thereof, W is aluminum, iron, titanium, gallium or mixtures thereof; c is 1 or 2; d is 2 when c is 1 or d is 3 or 5 when c is 2; M is an alkali metal cation, alkaline earth metal cation or mixtures thereof; n is the valence of M; and Q is a cation derived from 1-adamantamine, 3-quinuclidinol or 2-exo- aminonorbornane,
wherein the as-synthesized molecular sieve does not contain fluorine.

9. The molecular sieve of claim 8 wherein YO₂/W_{c}O_{d} is about 200-1500.

10. The molecular sieve of claim 8, wherein YO₂/W_{c}O_{d} is infinity.

11. A process for producing methylamine or dimethylamine comprising reacting methanol, dimethyl ether or a mixture thereof and ammonia in the gaseous phase in the presence of a catalyst comprising a molecular sieve prepared according to the method of any of claims 1 to 7 or a molecular sieve according to any of claims 8 to 10.

12. The process of claim 11 wherein the mole ratio of (1) an oxide selected from silicon oxide, germanium oxide or mixtures thereof to (2) an oxide selected from aluminum oxide, iron oxide, titanium oxide, gallium oxide or mixtures thereof is 200-1500.

13. The process of claim 11 wherein the methanol, dimethylether or mixture thereof and ammonia are present in amounts sufficient to provide a carbon/nitrogen ratio from about 0.2 to about 1.5.

14. The process of claim 11 conducted at a temperature of from about 250 °C to about 450 °C.

15. The process of claim 12 wherein the methanol, dimethylether or mixture thereof and ammonia are present in amounts sufficient to provide a carbon/nitrogen ratio from about 0.2 to about 1.5.

16. The process of claim 12 conducted at a temperature of from about 250 °C to about 450 °C.

17. A process for the production of light olefins from a feedstock comprising an oxygenate or mixture of oxygenates, the process comprising reacting the feedstock at effective conditions over a catalyst comprising a molecular sieve prepared according to the method of any of claims 1 to 7 or a molecular sieve according to any of claims 8 to 10.

18. The process of claim 17 wherein the mole ratio of (1) an oxide selected from silicon oxide, germanium oxide or mixtures thereof to (2) an oxide selected from aluminum oxide, iron oxide, titanium oxide, gallium oxide or mixtures thereof is 200-1500.

19. The process of claim 17 wherein the light olefins are ethylene, propylene, butylene or mixtures thereof.

20. The process of claim 18 wherein the light olefins are ethylene, propylene, butylene or mixtures thereof.

21. The process of claim 19 wherein the light olefin is ethylene.

22. The process of claim 20 wherein the light olefin is ethylene.

23. The process of claim 17 wherein the oxygenate is methanol, dimethyl ether or a mixture thereof.

24. The process of claim 18 wherein the oxygenate is methanol, dimethyl ether or a mixture thereof.

25. The process of claim 23 wherein the oxygenate is methanol.

26. The process of claim 24 wherein the oxygenate is methanol.

27. A process for separating gasses using a membrane containing a molecular sieve, the improvement comprising using as the molecular sieve a molecular sieve prepared according to the method of any of claims 1 to 7 or a molecular sieve according to any of claims 8 to 10.

28. The process of claim 27 wherein the mole ratio of (1) an oxide selected from silicon oxide, germanium oxide or mixtures thereof to (2) an oxide selected from aluminum oxide, iron oxide, titanium oxide, gallium oxide or mixtures thereof is 200-1500.

29. A process for the reduction of oxides of nitrogen contained in a gas stream wherein said process comprises contacting the gas stream with a molecular sieve prepared according to the method of any of claims 1 to 7 or a molecular sieve according to any of claims 8 to 10.

30. The process of claim 29 wherein the mole ratio of (1) an oxide selected from silicon oxide, germanium oxide or mixtures thereof to oxide (2) an oxide selected from aluminum oxide, iron oxide, titanium oxide, gallium oxide or mixtures thereof is 200-1500.

31. The process of claim 29 conducted in the presence of oxygen.

32. The process of claim 30 conducted in the presence of oxygen.

33. The process of claim 29 wherein said molecular sieve contains a metal or metal ions capable of catalyzing the reduction of the oxides of nitrogen.

34. The process of claim 30 wherein said molecular sieve contains a metal or metal ions capable of catalyzing the reduction of the oxides of nitrogen.

35. The process of claim 33 wherein the metal is cobalt, copper, platinum, iron, chromium, manganese, nickel, zinc, lanthanum, palladium, rhodium or mixtures thereof.

36. The process of claim 34 wherein the metal is cobalt, copper, platinum, iron, chromium, manganese, nickel, zinc, lanthanum, palladium, rhodium or mixtures thereof.

37. The process of claim 29 wherein the gas stream is the exhaust stream of an internal combustion engine.

38. The process of claim 30 wherein the gas stream is the exhaust stream of an internal combustion engine.

39. The process of claim 33 wherein the gas stream is the exhaust stream of an internal combustion engine.

40. The process of claim 34 wherein the gas stream is the exhaust stream of an internal combustion engine.

41. A process for treating a cold-start engine exhaust gas stream containing hydrocarbons and other pollutants consisting of flowing said engine exhaust gas stream over a molecular sieve bed which preferentially adsorbs the hydrocarbons over water to provide a first exhaust stream, and flowing the first exhaust gas stream over a catalyst to convert any residual hydrocarbons and other pollutants contained in the first exhaust gas stream to innocuous products and provide a treated exhaust stream and discharging the treated exhaust stream into the atmosphere, the molecular sieve bed **characterized in that** it comprises a molecular sieve prepared according to the method of any of claims 1 to 7 or a molecular sieve according to any of claims 8 to 10.

42. The process of claim 41 wherein the molecular sieve has a mole ratio of (1) an oxide selected from silicon oxide, germanium oxide or mixtures thereof to (2) an oxide selected from aluminum oxide, iron oxide, titanium oxide, gallium oxide or mixtures thereof of 200-1500.

43. The process of claim 41 wherein the oxides comprise silicon oxide and aluminum oxide.

44. The process of claim 41 wherein the oxides comprise silicon oxide and boron oxide.

45. The process of claim 41 wherein the molecular sieve comprises essentially all silicon oxide.

46. The process of claim 41 wherein the engine is an internal combustion engine.

47. The process of claim 46 wherein the internal combustion engine is an automobile engine.

48. The process of claim 41 wherein the engine is fueled by a hydrocarbonaceous fuel.

49. The process of claim 41 wherein the molecular sieve has deposited on it a metal selected from the group consisting of platinum, palladium, rhodium, ruthenium, and mixtures thereof.

50. The process of claim 49 wherein the metal is platinum.

51. The process of claim 48 wherein the metal is palladium.

52. The process of claim 48 wherein the metal is a mixture of platinum and palladium.

## Patentansprüche

1. Herstellungsverfahren für ein Molekularsieb mit der CHA-Kristallstruktur und einem Molverhältnis von größer als 200:1 von (1) Siliziumoxid, Germaniumoxid und deren Mischungen, zu (2) Aluminiumoxid, Eisenoxid, Titanoxid, Galliumoxid und deren Mischungen, das Verfahren umfassend:
A. Bilden einer wässrigen Reaktionsmischung, umfassend eine Zusammensetzung, die in Bezug auf Molverhältnisse, in die folgenden Bereiche fällt:
| | |
|---|---|
| YO₂ / WₐO_{b} | 350 bis ∞ |
| OH- / YO₂ | 0,19 bis 0,52 |
| Q / YO₂ | 0,15 bis 0,25 |
| M_{2/n}O / YO₂ | 0,04 bis 0,10 |
| H₂O / YO₂ | 10 bis 50 |
worin Y Silizium, Germanium oder deren Mischungen ist, W Aluminium, Eisen, Titan, Gallium oder deren Mischungen ist, a 1 oder 2 ist, b 2 ist, wenn a 1 ist oder b 3 ist, wenn a 2 ist; M ein Alkalimetall oder Erdalkalimetall ist, n die Valenz von M ist, und Q ein Kation ist, abgeleitet von 1-Adamantamin, 3-Quinuclidinol oder 2-exo-Aminonorbornan; und
B. Beibehalten der wässrigen Mischung unter ausreichenden Kristallisationsbedingungen, bis sich Kristalle gebildet haben,
worin der Molekularsieb in der Abwesenheit von Fluor hergestellt wird.

2. Verfahren gemäß Anspruch 1, worin die Reaktionsmischung zusätzlich Keime eines Molekularsiebs mit der CHA-Struktur umfasst.

3. Verfahren gemäß Anspruch 1, worin die Reaktionsmischung auf eine Temperatur von etwa 120 °C bis etwa 160 °C aufgeheizt wird.

4. Verfahren gemäß Anspruch 3, worin die Reaktionsmischung auf eine Temperatur von etwa 120 °C bis etwa 140 °C aufgeheizt wird.

5. Verfahren gemäß Anspruch 2, worin die Reaktionsmischung auf eine Temperatur von etwa 120 °C bis etwa 140 °C aufgeheizt wird.

6. Verfahren gemäß irgendeinem der Ansprüche 1 bis 5, worin das Molverhältnis von Oxid (1) zu Oxid (2) 200 bis 1500 ist.

7. Verfahren gemäß irgendeinem der Ansprüche 1 bis 6, worin das Molverhältnis von Oxid (1) zu Oxid (2) unendlich ist.

8. Molekularsieb mit der CHA-Kristallstruktur und einer Zusammensetzung, wie synthetisiert und im wasserfreien Zustand, in Bezug auf Molverhältnisse der Oxide, wie folgt:
| | |
|---|---|
| YO₂ / W_{c}O_{d} | 200 bis ∞ |
| M_{2/n}O / YO₂ | 0,04 bis 0,15 |
| Q / YO₂ | 0,15 bis 0,25 |
worin Y Silizium, Germanium oder deren Mischungen ist, W Aluminium, Eisen, Titan, Gallium oder deren Mischungen ist; c 1 oder 2 ist; d 2 ist, wenn c 1 ist oder d 3 oder 5 ist, wenn c 2 ist; M ein Alkalimetallkation ist, ein Erdalkalimetallkation oder deren Mischungen; n die Valenz von M ist; und Q ein Kation ist, abgeleitet von 1-Adamantamin, 3-Quinuclidinol oder 2-exo-Aminonorbornan,
worin das wie synthetisierte Molekularsieb kein Fluor enthält.

9. Molekularsieb gemäß Anspruch 8, worin YO₂ / W_{c}O_{d} etwa 200 bis 1500 ist.

10. Molekularsieb gemäß Anspruch 8, worin YO₂ / W_{c}O_{d} unendlich ist.

11. Herstellungsverfahren für Methylamin oder Dimethylamin, umfassend das Umsetzen von Methanol, Dimethylether oder einer Mischung davon, und Ammoniak im gasförmigen Zustand, in der Anwesenheit eines Katalysators, umfassend ein Molekularsieb, das gemäß dem Verfahren nach einem der Ansprüche von 1 bis 7 hergestellt wurde, oder ein Molekularsieb gemäß einem der Ansprüche 8 bis 10.

12. Verfahren gemäß Anspruch 11, worin das Molverhältnis von (1) einem Oxid, ausgewählt aus Siliziumoxid, Germaniumoxid oder deren Mischungen, zu (2) einem Oxid, ausgewählt aus Aluminiumoxid, Eisenoxid, Titanoxid, Galliumoxid oder deren Mischungen, von 200 bis 1500 ist.

13. Verfahren gemäß Anspruch 11, worin das Methanol, Dimethylether oder deren Mischung, und Ammoniak in einer Menge vorhanden sind, die ausreicht, ein Kohlenstoff-Stickstoff-Verhältnis von etwa 0,2 bis etwa 1,5 bereitzustellen.

14. Verfahren gemäß Anspruch 11, durchgeführt bei einer Temperatur von etwa 250 °C bis etwa 450 °C.

15. Verfahren gemäß Anspruch 12, worin das Methanol, Dimethylether oder deren Mischung, und Ammoniak in einer Menge vorhanden sind, die ausreicht, ein Kohlenstoff-Stickstoff-Verhältnis von etwa 0,2 bis etwa 1,5 bereitzustellen.

16. Verfahren gemäß Anspruch 12, durchgeführt bei einer Temperatur von etwa 250 °C bis etwa 450 °C.

17. Herstellungsverfahren für leichte Olefine aus einem Ausgangsmaterial, umfassend ein Oxygenat oder Mischung von Oxygenaten, das Verfahren umfassend das Umsetzen des Ausgangsmaterials unter wirksamen Bedingungen an einem Katalysator, umfassend ein Molekularsieb, hergestellt gemäß dem Verfahren nach irgendeinem der Ansprüche 1 bis 7 oder ein Molekularsieb gemäß irgendeinem der Ansprüche 8 bis 10.

18. Verfahren gemäß Anspruch 17, worin das Molverhältnis von (1) einem Oxid, ausgewählt aus Siliziumoxid, Germaniumoxid oder deren Mischungen, zu (2) einem Oxid, ausgewählt aus Aluminiumoxid, Eisenoxid, Titanoxid, Galliumoxid oder deren Mischungen, von 200 bis 1500 ist.

19. Verfahren gemäß Anspruch 17, worin die leichten Olefine Ethylen, Propylen, Butylen oder deren Mischungen sind.

20. Verfahren gemäß Anspruch 18, worin die leichten Olefine Ethylen, Propylen, Butylen oder deren Mischungen sind

21. Verfahren gemäß Anspruch 19, worin das leichte Olefin Ethylen ist.

22. Verfahren gemäß Anspruch 20, worin das leichte Olefin Ethylen ist.

23. Verfahren gemäß Anspruch 17, worin das Oxygenat Methanol, Diemthylether oder eine Mischung davon ist.

24. Verfahren gemäß Anspruch 18, worin das Oxygenat Methanol, Diemthylether oder eine Mischung davon ist.

25. Verfahren gemäß Anspruch 23, worin das Oxygenat Methanol ist.

26. Verfahren gemäß Anspruch 24, worin das Oxygenat Methanol ist.

27. Trennverfahren für Gase unter Verwendung einer Membran, die ein Molekularsieb enthält, die Verbesserung umfassend das Verwenden eines nach irgendeinem der Ansprüche 1 bis 7 hergestellten Molekularsiebs, als Molekularsieb oder ein Molekularsieb gemäß irgendeinem der Ansprüche 8 bis 10.

28. Verfahren gemäß Anspruch 27, worin das Molverhältnis von (1) einem Oxid, ausgewählt aus Siliziumoxid, Germaniumoxid oder deren Mischungen, zu (2) einem Oxid, ausgewählt aus Aluminiumoxid, Eisenoxid, Titanoxid, Galliumoxid oder deren Mischungen, von 200 bis 1500 ist.

29. Reduktionsverfahren für Stickoxide, die in einem Gasstrom enthalten sind, worin das Verfahren das Zusammenbringen des Gasstroms mit einem Molekularsieb umfasst, das gemäß dem Verfahren aus irgendeinem der Ansprüche 1 bis 7 hergestellt wurde, oder ein Molekularsieb nach irgendeinem der Ansprüche 8 bis 10.

30. Verfahren gemäß Anspruch 29, worin das Molverhältnis von (1) einem Oxid, ausgewählt aus Siliziumoxid, Germaniumoxid oder deren Mischungen, zu (2) einem Oxid, ausgewählt aus Aluminiumoxid, Eisenoxid, Titanoxid, Galliumoxid oder deren Mischungen, von 200 bis 1500 ist.

31. Verfahren gemäß Anspruch 29, durchgeführt in der Anwesenheit von Sauerstoff.

32. Verfahren gemäß Anspruch 30, durchgeführt in der Anwesenheit von Sauerstoff.

33. Verfahren gemäß Anspruch 29, worin das Molekularsieb ein Metall oder Metallionen enthält, die in der Lage sind, die Reduktion von Stickoxiden zu katalysieren.

34. Verfahren gemäß Anspruch 30, worin das Molekularsieb ein Metall oder Metallionen enthält, die in der Lage sind, die Reduktion von Stickoxiden zu katalysieren.

35. Verfahren gemäß Anspruch 33, worin das Metall Kobalt, Kupfer, Platin, Eisen, Chrom, Mangan, Nickel, Zink, Lanthan, Palladium, Rhodium oder deren Mischungen ist.

36. Verfahren gemäß Anspruch 34, worin das Metall Kobalt, Kupfer, Platin, Eisen, Chrom, Mangan, Nickel, Zink, Lanthan, Palladium, Rhodium oder deren Mischungen ist.

37. Verfahren gemäß Anspruch 29, worin der Gasstrom der Abgasstrom eines Verbrennungsmotors ist.

38. Verfahren gemäß Anspruch 30, worin der Gasstrom der Abgasstrom eines Verbrennungsmotors ist.

39. Verfahren gemäß Anspruch 33, worin worin der Gasstrom der Abgasstrom eines Verbrennungsmotors ist.

40. Verfahren gemäß Anspruch 34, worin der Gasstrom der Abgasstrom eines Verbrennungsmotors ist.

41. Behandlungsverfahren für einen Kaltstart-Motorabgasstrom, der Kohlenwasserstoffe und andere Schadstoffe enthält, bestehend aus dem Strömenlassen des Motorabgasstroms über ein Molekularsiebbett, das die Kohlenwasserstoffe bevorzugt an Wasser adsorbiert, um einen ersten Abgasstrom bereitzustellen, und dem Strömenlassen des ersten Abgasstroms über einen Katalysator, um alle restlichen Kohlenwasserstoffe und andere Verunreinigungen, die im ersten Abgasstrom enthalten sind, in harmlose Produkte umzuwandeln, und dem Bereitstellen eines behandelten Abgasstroms und dem Ausstoßen des behandelten Abgasstroms in die Atmosphäre, wobei das Molekularsiebbett dadurch charakterisiert ist, dass es ein Molekularsieb umfasst, das gemäß dem Verfahren aus irgendeinem der Ansprüche 1 bis 7 hergestellt wurde, oder ein Molekularsieb gemäß irgendeinem der Ansprüche 8 bis 10.

42. Verfahren gemäß Anspruch 41, worin der Molekularsieb ein Molverhältnis von (1) einem Oxid, ausgewählt aus Siliziumoxid, Germaniumoxid oder deren Mischungen, zu (2) einem Oxid, ausgewählt aus Aluminiumoxid, Eisenoxid, Titanoxid, Galliumoxid oder deren Mischungen, von 200 bis 1500 hat.

43. Verfahren gemäß Anspruch 41, worin die Oxide Siliziumoxid und Aluminiumoxid umfassen.

44. Verfahren gemäß Anspruch 41, worin die Oxide Siliziumoxid und Boroxid umfassen.

45. Verfahren gemäß Anspruch 41, worin das Molekularsieb im Wesentlichen alles Siliziumoxid umfasst.

46. Verfahren gemäß Anspruch 41, worin der Motor ein Verbrennungsmotor ist.

47. Verfahren gemäß Anspruch 46, worin der Verbrennungsmotor ein Automobilmotor ist.

48. Verfahren gemäß Anspruch 41, worin der Motor mit einem kohlenwasserstoffhaltigem Kraftstoff betrieben wird.

49. Verfahren gemäß Anspruch 41, worin auf dem Molekularsieb ein Metall abgeschieden wurde, ausgewählt aus der Gruppe Platin, Palladium, Rhodium, Ruthenium und deren Mischungen.

50. Verfahren gemäß Anspruch 49, worin das Metall Platin ist.

51. Verfahren gemäß Anspruch 48, worin das Metall Palladium ist.

52. Verfahren gemäß Anspruch 48, worin das Metall eine Mischung aus Platin und Palladium ist.

## Revendications

1. Un procédé de préparation pour un tamis moléculaire ayant la structure cristalline CHA et un rapport molaire supérieur à 200:1 de (1) l'oxyde de silicium, l'oxyde de germanium et leurs mélanges par rapport à (2) l'oxyde d'aluminium, l'oxyde de fer, l'oxyde de titane, l'oxyde gallium et leurs mélanges, comprenant ledit procédé:
A. former un mélange réactionnel aqueux comprenant une composition en termes de rapports molaires, dans les gammes suivantes:
| | |
|---|---|
| YO₂ / WₐO_{b} | 350 à ∞ |
| OH- / YO₂ | 0,19 à 0,52 |
| Q / YO₂ | 0,15 à 0,25 |
| M_{2/n}O / YO₂ | 0,04 à 0,10 |
| H₂O/ YO₂ | 10 à 50 |
dans lequel Y est le silicium, le germanium ou leurs mélanges, W est l'aluminium, le fer, le titane, le gallium ou leurs mélanges, a est 1 ou 2, b est 2, si a est 1 ou b est 3, si a est 2; M est un métal alcalin ou un métal alcalin-terreux, n est la valence de M, et Q est un cation dérivé de 1-adamantamine, 3-quinuclidinol ou 2-exo-aminonorbornane; et
B. maintenir ledit mélange aqueux dans des conditions de cristallisation suffisantes jusqu'à la formation de cristaux,
dans lequel le tamis moléculaire est préparé en l'absence de fluor.

2. Le procédé selon la revendication 1, dans lequel le mélange réactionnel comprend en outre des germes d'un tamis moléculaire ayant la structure CHA.

3. Le procédé selon la revendication 1, dans lequel le mélange réactionnel est chauffé à une température d'environ 120 °C à environ 160 °C.

4. Le procédé selon la revendication 3, dans lequel le mélange réactionnel est chauffé à une température d'environ 120 °C à environ 140 °C.

5. Le procédé selon la revendication 2, dans lequel le mélange réactionnel est chauffé à une température d'environ 120 °C à environ 140 °C.

6. Le procédé selon l'une quelconque des revendications 1 à 5, dans lequel le rapport molaire de l'oxyde (1) par rapport à l'oxyde (2) est 200 à 1500.

7. Le procédé selon l'une quelconque des revendications 1 à 6, dans lequel le rapport molaire de l'oxyde (1) par rapport à l'oxyde (2) est infini.

8. Un tamis moléculaire ayant la structure cristalline CHA et ayant une composition telle que synthétisée et à l'état anhydre, en termes de rapports molaires d'oxydes comme suit:
| | |
|---|---|
| YO₂ / W_{c}O_{d} | 200 à ∞ |
| M_{2/n}O / YO₂ | 0,04 à 0,15 |
| Q / YO₂ | 0,15 à 0,25 |
dans lequel Y est le silicium, le germanium ou leurs mélanges, W est l'aluminium, le fer, le titane, le gallium ou leurs mélanges; c est 1 ou 2; d est 2, si c est 1 ou d est 3 ou 5, si c est 2; M est un cation de métal alcalin, un cation de métal alcalin-terreux ou leurs mélanges; n est la valence de M; et Q est un cation dérivé de 1-adamantamine, de 3-quinuclidinole ou de 2-exo-aminonorbornane,
dans lequel le tamis moléculaire tel que synthétisé ne contient pas de fluor.

9. Le tamis moléculaire selon la revendication 8, dans lequel YO₂ / W_{c}O_{d} est environ 200 à 1500.

10. Le tamis moléculaire selon la revendication 8, dans lequel YO₂ / W_{c}O_{d} est infini.

11. Un procédé de production de méthylamine ou diméthylamine comprenant la réaction du méthanol, de l'éther diméthylique ou d'un mélange de ceux-ci et d'ammoniac en phase gazeuse, en présence d'un catalyseur comprenant un tamis moléculaire préparé selon le procédé de l'une quelconque des revendications 1 à 7, ou un tamis moléculaire selon l'une quelconque des revendications 8 à 10.

12. Le procédé selon la revendication 11, dans lequel le rapport molaire de (1) un oxyde choisi parmi l'oxyde de silicium, l'oxyde de germanium ou leurs mélanges par rapport à (2) un oxyde choisi parmi l'oxyde d'aluminium, l'oxyde de fer, l'oxyde de titane, l'oxyde de gallium ou leurs mélanges, est 200 à 1500.

13. Le procédé selon la revendication 11, dans lequel le méthanol, l'éther diméthylique ou le mélange de ceux-ci et ammoniac sont présents en quantités suffisantes pour fournir un rapport carbone / azote d'environ 0,2 à environ 1,5.

14. Le procédé selon la revendication 11, conduit à une température d'environ 250 °C à environ 450 °C.

15. Le procédé selon la revendication 12, dans lequel le méthanol, l'éther diméthylique ou le mélange de ceux-ci et l'ammoniac sont présents en quantités suffisantes pour fournir un rapport carbone / azote d'environ 0,2 à environ 1,5.

16. Le procédé selon la revendication 12, conduit à une température d'environ 250 °C à environ 450 °C.

17. Un procédé de production pour oléfines légères à partir d'une première charge d'alimentation comprenant un oxygénat or un mélange des oxygénats, le procédé comprenant la réaction de la charge d'alimentation dans des conditions efficaces sur un catalyseur comprenant un tamis moléculaire préparé selon le procédé de l'une quelconque des revendications 1 à 7 ou un tamis moléculaire selon l'une quelconque des revendications 8 à 10.

18. Le procédé selon la revendication 17, dans lequel le rapport molaire de (1) un oxyde choisi parmi l'oxyde de silicium, l'oxyde de germanium ou leurs mélanges par rapport à (2) un oxyde choisi parmi l'oxyde d'aluminium, l'oxyde de fer, l'oxyde de titane, l'oxyde de gallium ou leurs mélanges, est 200 à 1500.

19. Le procédé selon la revendication 17, dans lequel les oléfines légères sont l'éthylène, le propylène, le butylène ou leurs mélanges.

20. Le procédé selon la revendication 18, dans lequel les oléfines légères sont l'éthylène, le propylène, le butylène ou leurs mélanges.

21. Le procédé selon la revendication 19, dans lequel l'oléfine légère est l'éthylène.

22. Le procédé selon la revendication 20, dans lequel l'oléfine légère est l'éthylène.

23. Le procédé selon la revendication 17, dans lequel l'oxygénat est le méthanol, l'éther diméthylique ou un mélange de ceux-ci.

24. Le procédé selon la revendication 18, dans lequel l'oxygénat est le méthanol, l'éther diméthylique ou un mélange de ceux-ci.

25. Le procédé selon la recendication 23, dans lequel l'oxygénat est le méthanol.

26. Le procédé selon la revendication 24, dans lequel l'oxygénat est le méthanol.

27. Un procédé de séparation de gaz utilisant une membrane contenant un tamis moléculaire, l'amélioration comprenant l'utilisation comme tamis moléculaire d'un tamis moléculaire préparé selon le procédé de l'une quelconque des revendications 1 à 7 ou d'un tamis moléculaire selon l'une quelconque des revendications 8 à 10.

28. Le procédé selon la revendication 27, dans lequel le rapport molaire de (1) un oxyde choisi parmi l'oxyde de silicium, l'oxyde de germanium ou leurs mélanges par rapport à (2) un oxyde choisi parmi l'oxyde d'aluminium, l'oxyde de fer, l'oxyde de titane, l'oxyde de gallium ou leurs mélanges, est 200 à 1500.

29. Un procédé de réduction des oxydes d'azote contenus dans un flux de gaz, dans lequel ledit procédé comprend la mise en contact du flux de gaz avec un tamis moléculaire préparé selon le procédé de l'une quelconque des revendications 1 à 7 ou un tamis moléculaire selon l'une quelconque des revendications 8 à 10.

30. Le procédé selon la revendication 29, dans lequel le rapport molaire de (1) un oxyde choisi parmi l'oxyde de silicium, l'oxyde de germanium ou leurs mélanges, par rapport à (2) un oxyde choisi parmi l'oxyde d'aluminium, l'oxyde de fer, l'oxyde de titane, l'oxyde de gallium ou leurs mélanges, est 200 à 1500.

31. Le procédé selon la revendication 29, conduit en présence d'oxygène.

32. Le procédé selon la revendication 30, conduit en présence d'oxygène.

33. Le procédé selon la revendication 29, dans lequel ledit tamis moléculaire contient un métal ou des ions métalliques, capables de catalyser la réduction des oxydes d'azote.

34. Le procédé selon la revendication 30, dans lequel ledit tamis moléculaire contient un métal ou des ions métalliques, capables de catalyser la réduction des oxydes d'azote.

35. Le procédé selon la revendication 33, dans lequel le métal est le cobalt, le cuivre, le platine, le fer, le chrome, le manganèse, le nickel, le zinc, le lanthane, le palladium, le rhodium ou leurs mélanges.

36. Le procédé selon la revendication 34, dans lequel le métal est le cobalt, le cuivre, le platine, le fer, le chrome, le manganèse, le nickel, le zinc, le lanthane, le palladium, le rhodium ou leurs mélanges.

37. Le procédé selon la revendication 29, dans lequel le flux de gaz est le flux d'échappement d'un moteur à combustion interne.

38. Le procédé selon la revendication 30, dans lequel le flux de gaz est le flux d'échappement d'un moteur à combustion interne.

39. Le procédé selon la revendication 33, dans lequel le flux de gaz est le flux d'échappement d'un moteur à combustion interne.

40. Le procédé selon la revendication 34, dans lequel le flux de gaz est le flux d'échappement d'un moteur à combustion interne.

41. Un procédé de traitement d'un flux de gaz d'échappement de moteur à démarrage à froid contenant des hydrocarbures et d'autres polluants, constitué en faire circuler ledit flux de gaz d'échappement du moteur sur un lit de tamis moléculaire qui adsorbe préférentiellement les hydrocarbures sur l'eau pour fournir un premier flux d'échappement, et faire circuler le premier flux de gaz d'échappement sur un catalyseur pour convertir tous les hydrocarbures résiduels et les autres polluants contenus dans le premier flux de gaz d'échappement en produits inoffensifs et fournir un flux d'échappement traité et rejeter le flux d'échappement traité dans l'atmosphère, le lit de tamis moléculaire **caractérisé en ce qu'**il comprend un tamis moléculaire préparé selon le procédé de l'une quelconque des revendications 1 à 7 ou un tamis moléculaire selon l'une quelconque des revendication 8 à 10.

42. Le procédé selon la revendication 41, dans lequel le tamis moléculaire a un rapport molaire de (1) un oxyde choisi parmi l'oxyde de silicium, l'oxyde de germanium ou leurs mélanges par rapport à (2) un oxyde choisi parmi l'oxyde d'aluminium, l'oxyde de fer, l'oxyde de titane, l'oxyde de gallium ou leurs mélanges, de 200 à 1500.

43. Le procédé selon la revendication 41, dans lequel les oxydes comprennent l'oxyde de silicium et l'oxyde d'aluminium.

44. Le procédé selon la revendication 41, dans lequel les oxydes comprennent l'oxyde de silicium et l'oxyde de bore.

45. Le procédé selon la revendication 41, dans lequel le tamis moléculaire comprend essentiellement tout l'oxyde de silicium.

46. Le procédé selon la revendication 41, dans lequel le moteur est un moteur à combustion interne.

47. Le procédé selon la revendication 46, dans lequel le moteur à combustion interne est un moteur automobile.

48. Le procédé selon la revendication 41, dans lequel le moteur est alimenté par un carburant hydrocarboné.

49. Le procédé selon la revendication 41, dans lequel le tamis moléculaire est recouvert d'un métal choisi parmi le groupe constitué en le platine, le palladium, le rhodium, le ruthénium et leurs mélanges.

50. Le procédé selon la revendication 49, dans lequel le métal est le platine.

51. Le procédé selon la revendication 48, dans lequel le métal est le palladium.

52. Le procédé selon la revendication 48, dans lequel le métal est un mélange de platine et de pallidium.
